(19) Europäisches Patentamt
European Patent Office
Office européen des brevets

(11) Veröffentlichungsnummer: **0 057 425**
**A1**

(12) # EUROPÄISCHE PATENTANMELDUNG

(21) Anmeldenummer: **82100568.3**

(51) Int. Cl.³: **C 07 D 311/58**, C 08 K 5/15

(22) Anmeldetag: **28.01.82**

(30) Priorität: **04.02.81 DE 3103740**

(43) Veröffentlichungstag der Anmeldung: **11.08.82**
**Patentblatt 82/32**

(84) Benannte Vertragsstaaten: **BE CH DE FR GB IT LI NL**

(71) Anmelder: **BASF Aktiengesellschaft,**
**Carl-Bosch-Strasse 38, D-6700 Ludwigshafen (DE)**

(72) Erfinder: **Horner, Michael, Dr., Im Ritterbueschel 9,**
**D-6730 Neustadt (DE)**
Erfinder: **Teege, Gernot, Dr., Alwin-Mittasch-Platz 12,**
**D-6700 Ludwigshafen (DE)**

(54) **Chromanderivate.**

(57) Die Erfindung betrifft neue Chromanderivate

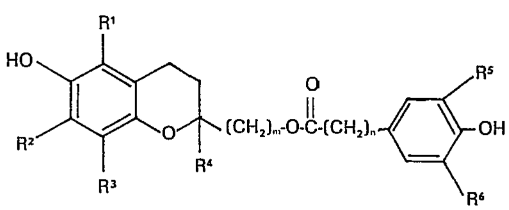

I

($R^1$-$R^4$ = H, $C_1$-$C_4$-Alkyl; $R^5$ = sek.- oder tert.-$C_1$-$C_8$-Alkyl; $R^6$ = H, $R^5$; m = 1, 2 oder 3; n = 0, 1, 2 oder 3), ein Verfahren zur Herstellung dieser Verbindungen sowie deren Anwendung als Stabilisatoren für Kunststoffe.

EP 0 057 425 A1

Chromanderivate

Die vorliegende Erfindung betrifft neue Chromanderivate der allgemeinen Formel I

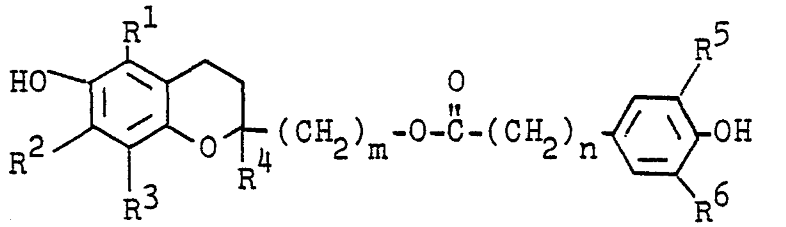

I

In dieser Formel bedeuten

| | |
|---|---|
| $R^1$ - $R^4$ | H; $C_1$-$C_4$-Alkyl |
| $R^5$ | sek.- oder tert.-$C_1$-$C_8$-Alkyl |
| $R^6$ | H; $R^5$ |
| m | 1, 2 oder 3 |
| n | 0, 1, 2 oder 3 |

Außerdem betrifft die Erfindung die Herstellung der Verbindungen I, deren Verwendung als Stabilisator von Kunststoffen gegen die Einflüsse von Licht, Wärme und Oxidationsmitteln sowie die mit diesen Verbindungen stabilisierten Kunststoffe.

Chromanderivate, darunter vor allem das α-Tocopherol (Vitamin E)

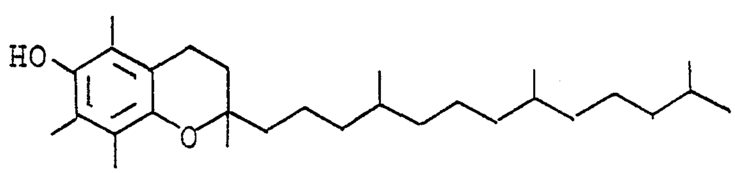

sind allgemein als Stabilisatoren für Kunststoffe und organische Massen bekannt (vgl. DE-PS 11 14 319 und 11 36 102).

Mi/P

Obwohl das α-Tocopherol eine gute Stabilisierungswirkung gegen den Abbau von Kunststoffen bei mehrfacher Wiederverarbeitung durch jeweiliges Wiederaufschmelzen der Kunststoffe zeigt, läßt es in anderer Hinsicht zu wünschen übrig. Häufig gibt es zu Verfärbungen Anlaß, und im Ofenalterungstest, aus welchem sich auf die Lebensdauer des Kunststoffs schließen läßt, liefert es nur unbefriedigende Stabilisierungswerte. Auch in verarbeitungstechnischer Hinsicht bietet die oxidationsempfindliche ölige Substanz α-Tocopherol Probleme. Außerdem ist das α-Tocopherol verhältnismäßig teuer, so daß es als Zusatz zu Massenprodukten wie Kunststoffen aus wirtschaftlichen Gründen nur ausnahmsweise in Betracht kommt.

Einfachere Chromanderivate wie diejenigen gemäß der DE-OS 23 64 141 sind zwar billiger, dafür aber auch insgesamt weniger wirksam.

Allgemein gilt, daß bestimmte Stabilisatoren meist nur in einigen Stabilisierungseigenschaften gut geeignet sind, in anderer Hinsicht dagegen Mängel aufweisen. Beispielsweise bieten die handelsüblichen phenolischen Stabilisatoren wie Pentaerythrityl-tetrakis-[3-(3,5-di-tert.-butyl-4-hydroxyphenyl)]-propionat guten Schutz gegen den oxidativen Abbau der Kunststoffe, nicht aber gegen den Abbau der Kunststoffe bei ihrer Verarbeitung und insbesondere mehrfacher Wiederverarbeitung.

Aus diesem Grunde wurden bisher häufig Mischungen verschiedener Stabilisatoren verwendet, wodurch sich indes meistens verfahrenstechnische Schwierigkeiten ergeben.

Der Erfindung lag daher, wie im Falle der älteren Patentanmeldung DE-OS 30 10 505, die Aufgabe zugrunde, den genannten Nachteilen abzuhelfen und der Technik billigere

Stabilisierungsmittel mit einem breiteren Spektrum der gewünschten stabilisierenden Eigenschaften zur Verfügung zu stellen.

Demgemäß wurde gefunden, daß sich die eingangs definierten Chromanderivate I hervorragend als Stabilisierungsmittel für Kunststoffe eignen.

Ferner wurden verschiedene Verfahren zur Herstellung der Chromanderivate I gefunden, die im folgenden näher beschrieben werden.

Unter den Verbindungen I werden solche bevorzugt, in denen die Reste $R^1$ - $R^4$ Methylgruppen bedeuten, da Chromanderivate dieser Struktur besonders leicht zugänglich sind. Verbindungen I mit anderen Resten $R^1$ - $R^4$ als jeweils der Methylgruppe sind in analoger Weise zugänglich und ihre Wirkung als Stabilisatoren ist etwa die gleiche wie bei den Tetramethylchromanderivaten.

Wesentlich für die Stabilisatorwirkung ist der in Bezug auf die phenolische Hydroxylgruppe orthoständige Substituent $R^5$. Grundsätzlich kommen alle definitionsgemäßen sekundären und tertiären Alkylgruppen in Betracht, jedoch bevorzugt man aus wirtschaftlichen Gründen Verbindungen, in denen $R^5$ die Isopropyl- oder vor allem die tert.-Butylgruppe ist. Weiterhin werden solche Verbindungen wegen ihres meist besonders starken stabilisierenden Effektes bevorzugt, in denen auch der Rest $R^6$ für einen der Reste $R^5$ steht. Die Anzahl der Methylengruppen m bzw. n hat hingegen keinen wesentlichen Einfluß auf die stabilisierende Wirkung der Verbindungen I.

Die Chromanderivate I sind in üblicher Weise durch Veresterung der Chromanderivate II

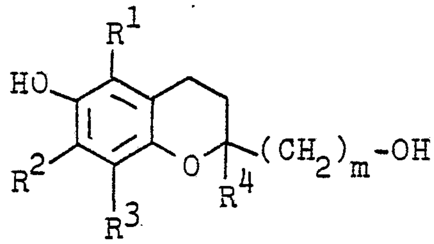
II

mit den Phenolderivaten der allgemeinen Formel III

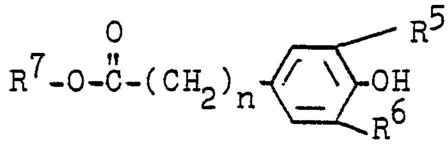
III

in der $R^7$ für Wasserstoff oder einen $C_1$-$C_4$-Alkylrest steht, erhältlich. Die Veresterung ($R^7$=H) bzw. Umesterung ($R^7$ = $C_1$-$C_4$-Alkyl) ist in ihren zahlreichen Ausgestaltungen hinsichtlich der sauren oder basischen Katalysatoren, der Entfernung des Reaktionswassers bzw. der abgespaltenen Alkohole, der Temperatur und der Reaktionszeit allgemein bekannt, so daß sich nähere Ausführungen hierüber erübrigen.

Die Reinigung der Verbindungen I kann, falls überhaupt erforderlich, durch Umkristallisation, z.B. aus Methanol/Wasser, erfolgen.

Die Verbindungen II sowie deren Herstellung sind aus der DE-OS 30 10 504 bekannt.

Die Phenolderivate III sind größtenteils ebenfalls bekannt (vgl. z.B. DE-PS 1 201 349) und können allgemein in bekannter Weise z.B. durch Addition von Acrylverbindungen IV an Phenole V

$R^7-O-\overset{O}{\overset{\|}{C}}-CH=CH_2$ + 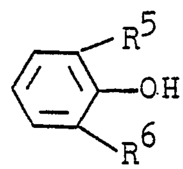 III

hergestellt werden.

Die erfindungsgemäßen Chromanderivate I eignen sich hervorragend als Stabilisatoren von Kunststoffen gegen Schädigung durch Wärme, Licht und oxidative Einflüsse. Besonders hervorzuheben sind die stabilisierenden Eigenschaften der Chromanderivate I in empfindlichen Kunststoffen wie Polypropylen, Polyisobutylen, Styrol und Acrylester/Butandien/Styrol-Polymerisaten. Je nach Beanspruchung dieser Materialien verwendet man die Stabilisatoren in Konzentrationen von 0,005 bis 1,0, in der Regel von 0,01 bis 0,5 Gew.%, bezogen auf die Menge des Kunststoffs.

Die erfindungsgemäßen Stabilisatoren können allein oder in Mischung mit anderen Stabilisatoren, besonders mit sogenannten Synergisten, verwendet werden. Synergisten sind solche Verbindungen, die für sich allein keine oder nur geringe stabilisierende Wirkung haben, zusammen mit Stabilisierungsmitteln deren Effekt jedoch deutlich verbessern. Solche Synergisten sind beispielsweise Calciumstearat und Distearylthiodipropionat IV

$$S-(CH_2-CH_2\overset{O}{\overset{\|}{C}}O\ \text{stearyl})_2 \qquad \text{IV}$$

Man verwendet die Synergisten im allgemeinen in Mengen von 50 bis 500 Gew.%, bezogen auf die Menge des Stabilisierungsmittels. Der besondere Vorteil der erfindungsgemäßen Stabilisatoren ist es jedoch, daß die Mitverwendung von anderen Stabilisatoren oder Synergisten meistens entbehrlich ist.

Für die Eignung und Wirksamkeit von Stabilisatoren sind vor allem folgende Kriterien maßgebend:

1. Farbe
Das Substrat soll durch den Stabilisator nicht verfärbt werden. Diese Forderung, die naturgemäß für farblose Kunststoffe von besonderer Wichtigkeit ist, wird durch die erfindungsgemäßen Stabilisatoren bei den meisten Kunststoffen befriedigend bis ausgezeichnet erfüllt, und in aller Regel sind diese Verbindungen herkömmlichen Stabilisierungsmitteln, darunter auch dem $\alpha$-Tocopherol, überlegen. Die quantitative Ermittlung der Farbeigenschaften kann nach verschiedenen Methoden bestimmt werden, z.B. nach dem Yellowness-Test (ASTMD 1925).

2. Verarbeitungsstabilität
Hierunter ist die Eigenschaftskonstanz von Thermoplasten gegenüber der mechanischen und thermischen Beanspruchung bei Formgebungsverfahren wie der Extrusion und dem Spritzguß zu verstehen. In dieser Beziehung werden mit den erfindungsgemäßen Stabilisatoren besonders gute Ergebnisse erzielt. Eine Maßzahl für die Verarbeitungsstabilität läßt sich aus der Änderung des Schmelzverhaltens des betreffenden Thermoplasten nach mehrmaliger Formgebung unter Wiederaufschmelzen ableiten. Der entsprechende Schmelzindex-Test ist in DIN 53 735 beschrieben. Ein wichtiges Kriterium für die Verarbeitungsstablität ist auch die Farbkonstanz, die z.B. nach dem Yellowness-Test ermittelt werden kann.

3. Langzeitstabilität
Das Verhalten des Kunststoffes gegen rigorose thermische und oxidative Einflüsse ist ein Indiz für die

Dauer der Qualitätskonstanz bei bestimmungsgemäßem Gebrauch, d.h. aus den bei den entsprechenden Tests (DIN 53 383, Blatt 1) ermittelten Werten läßt sich die Lebensdauer des Kunststoffartikels abschätzen. Hier bieten die erfindungsgemäßen Stabilisatoren in Verbindung mit Synergisten Vorteile.

Nähere Angaben zur Qualitätsprüfung der erfindungsgemäßen Stabilisatoren sind den Versuchen über deren Wirkung zu entnehmen.

Beispiele für die Herstellung von Chromanderivaten I

Beispiel 1

6-Hydroxy-2,5,7,8-tetramethyl-2-[3-oxa-4-oxo-7-(3,5-di--tert.-butyl-4-hydroxy)-phenyl]-hept-1-yl-chroman

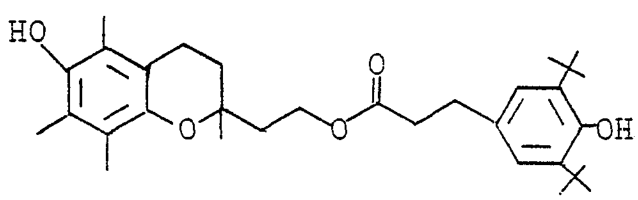

In einer Rührapparatur wurde eine Lösung aus 20 g (0,077 mol) 3-(3,5-Di-tert.-butyl-4-hydroxyphenyl)-propionsäure, 19,2 g (0,077 mol) 6-Hydroxy-2,5,7,8-tetramethyl-2--(2-hydroxyethyl)-chroman und 0,2 g p-Toluolsulfonsäure und 200 ml Toluol 19 h unter Rückflußkühlung erhitzt. Die übliche Aufarbeitung des Reaktionsgemisches lieferte das oben genannte Verfahrensprodukt nach Umkristallisation aus Methanol/Wasser als farblose Kristalle in 69%iger Ausbeute; Smp. 95 - 98°C.

Nach dem Umesterungsverfahren, ausgehend von dem entsprechenden Propionsäuremethylester, fiel die gleiche Verbindung in 80%iger Ausbeute an.

Beispiel 2

6-Hydroxy-2,5,7,8-tetramethyl-2-[2-oxa-3-oxo-6-(3,5-di-tert.-butyl-4-hydroxy)-phenyl]-hex-1-yl-chroman.

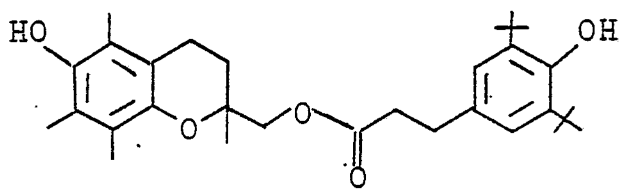

Analog Beispiel 1 wurde die oben genannte Verbindung aus dem 2-Hydroxymethylchroman sowie dem dort genannten Phenylpropionsäurederivat in 48%iger Ausbeute hergestellt. Langsam kristallisierendes bräunliches Öl.

Versuche zur stabilisierenden Wirkung der Chromanderivate I

1. Farbe von Polypropylen
Hier wurde die Farbqualität als Yellowness-Index YI nach dem Yellowness-Test (ASTMD 1925) gemessen.

Als Testmaterial diente additivfreies dechloriertes Polypropylen, in welches der Stabilisator auf jeweils gleiche Weise eingearbeitet wurde, und das zu Granulat von 15 mm Schichtdicke und Platten von 1 mm Schichtdicke geformt wurde. Die angegebenen YI-Werte sind jeweils das Mittel aus zwei Messungen. Je höher diese Werte, umso geringer ist die Farbqualität. Die Ergebnisse sind der Tabelle zu entnehmen. Den Werten entsprechen etwa folgende wahrnehmbare Verfärbungen des Testmaterials:

| | |
|---|---|
| 2 | nicht erkennbare Verfärbung |
| 3-5 | sehr schwache Verfärbung |
| 5-10 | schwache, aber bereits deutlich erkennbare Verfärbung |
| 10-20 | merkliche Verfärbung |
| 20 | starke Verfärbung |

2. Verarbeitungsstabilität von Polypropylen
Die Polypropylenproben (gleiches Material wie beim Farbtest) wurden sechsmal extrudiert und granuliert. Aus den Schmelzindices MFI (zur Bestimmungsmethode s. DIN 53 735) nach der ersten und der sechsten Extrusion wurde der Quotient $MFI_6/MFI_1$ gebildet. Je größer dieser Quotient ist, umso geringer ist die Verarbeitungsstabilität. Die Farbmessungen entsprechen dem vom Farbtest. Die Ergebnisse gehen ebenfalls aus der Tabelle hervor.

3. Langzeitstailität von Polypropylen
Polypropylenplatten der Beschaffenheit wie beim Farbtest wurden nach DIN 53 383, Blatt 1, der sogenannten Ofenalterung unterworfen, indem die Platten im Wärmeschrank unter Frischluftzufuhr solange auf 140°C erhitzt wurden, bis sie erkennbar versprödeten. Die visuelle Prüfung wurde alle 24 Stunden vorgenommen, d.h. die Alterung wurde hier in Tagen gemessen. Je geringer die Werte, desto geringer die Langzeitstabilität. Die Werte sind Mittelwerte aus zehn Messungen und verstehen sich jeweils mit einer Abweichung mit bis zu etwa 5 %. Die Ergebnisse sind in der Tabelle zusammengestellt.

Tabelle

| Vers. Nr. | Stabilisator | gemäß Bsp. Nr. | Menge Gew.% | Farbe YI-Index nach Einarb. | Verarbeitungsstabilität $MFI_6/MFI_1$ | $\Delta YI$ $(=YI_6-YI_1)$ | Langzeitstabilität (h) |
|---|---|---|---|---|---|---|---|
| | zum Vergleich | | | | | | |
| 1 | ohne Stabilisator | - | - | 1 | 7,3 | 4 | < 24 |
| 2 | Q x) | - | 0,1 | 8 | 2,5 | 13 | 707 |
| 3 | α-Tocopherol | - | 0,1 | 18 | 1,9 | 10 | 70 |
| 4 | α-Tocopherol | | 0,1 | | | | |
| | Q | | 0,5 | 15 | 1,5 | 11 | 215 |
| | erfindungsgemäß | | | | | | |
| 5 | 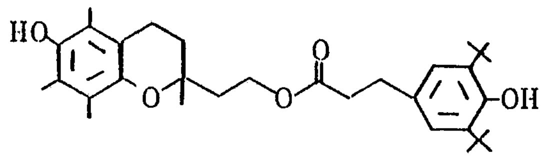 | 1 | 0,1 | 13 | 1,3 | 18 | 314 |
| 6 | 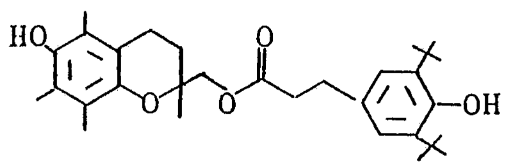 | 2 | 0,1 | 12 | 1,2 | 14 | 314 |

x) Neopentylglykol- 3-(3,5-di-tert.butyl-4-hydroxyphenyl)-propionsäure -tetraester, handelsüblich

## Patentansprüche

1. Chromanderivate der allgemeinen Formel I

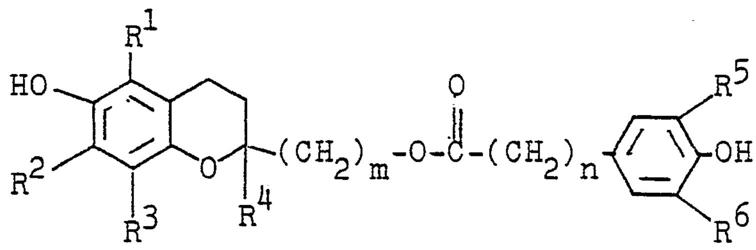

I

in welcher die Substituenten und Indices folgende Bedeutung haben:

| | |
|---|---|
| $R^1$ - $R^4$ | H; $C_1$-$C_4$-Alkyl |
| $R^5$ | sek.- oder tert.-$C_1$-$C_8$-Alkyl |
| $R^6$ | H; $R^5$ |
| m | 1, 2 oder 3 |
| n | 0, 1, 2 oder 3. |

2. Verfahren zur Herstellung der Chromanderivate I, dadurch gekennzeichnet, daß man ein Chromanderivat II

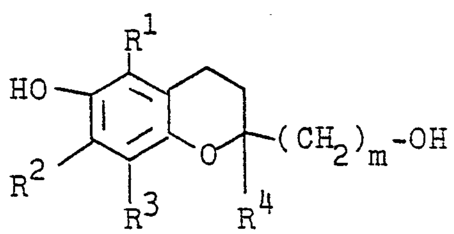

II

in an sich bekannter Weise mit einem Phenolderivat der allgemeinen Formel III

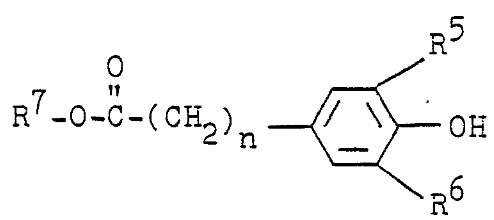

III

in welcher $R^7$ Wasserstoff oder eine $C_1$-$C_4$-Alkylgruppe bedeutet, umsetzt.

3. Verwendung der Chromanderivate I zum Stabilisieren von Kunststoffen in Konzentrationen von 0,01 bis 5 Gew.%, bezogen auf die Menge des Kunststoffs.

4. Kunststoffe, gekennzeichnet durch einen Gehalt von 0,01 bis 5 Gew.%, bezogen auf die Menge des Kunststoffes, eines Chromanderivates I.

0057425

Europäisches Patentamt

# EUROPÄISCHER RECHERCHENBERICHT

Nummer der Anmeldung

EP 82 10 0568.3

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| P,A | EP - A1 - 0 036 169 (BASF)<br>* Ansprüche 1, 5, 10 * | 1-3 | C 07 D 311/58<br>C 08 K 5/15 |
| D,P,A | & DE - A1 - 3 010 505<br>-- | | |
| D,A | DE - A - 2 364 141 (F. HOFFMANN-LA ROCHE & CO.)<br>* Ansprüche 25, 38 *<br>-- | 1,3 | |
| | | | RECHERCHIERTE SACHGEBIETE (Int. Cl.³) |
| A | Patent Abstracts of Japan<br>Band 2, Nr. 117, 29. September 1978<br>Seite 2187C78<br>& JP - A - 53 - 78248<br>-- | 3 | |
| A | Patent Abstracts of Japan<br>Band 3, Nr. 46, 18. April 1979<br>Seite 31C43<br>& JP - A - 54 - 20056<br>---- | 3 | C 07 D 311/58<br>C 08 K 5/15 |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 22-04-1982 | FROELICH |